# EUROPEAN PATENT SPECIFICATION

(11) **EP 1 933 893 B1**
(45) Date of publication and mention of the grant of the patent: **24.02.2010**
(21) Application number: 06806222.3
(22) Date of filing: 12.10.2006
(51) Int. Cl.: A61L 31/08, A61L 31/16

(54) **IMPLANT WITH MULTIPLE COATING**
IMPLANTAT MIT MEHRFACHBESCHICHTUNG
IMPLANT À REVÊTEMENT MULTIPLE

(30) Priority: 12.10.2005 EP 05022217; 12.10.2005 US 725319 P
(43) Date of publication of application: 25.06.2008
(73) Proprietor: Schömig, Albert, 80638 München (DE); Wessely, Rainer, 40489 Düsseldorf (DE); Kastrati, Adnan, 81925 München (DE)
(72) Inventor: Schömig, Albert, 80638 München (DE); Wessely, Rainer, 40489 Düsseldorf (DE); Kastrati, Adnan, 81925 München (DE)
(74) Representative: Wichmann, Hendrik
(86) International application number: PCT/EP2006/009864
(87) International publication number: WO 2007/042294

(56) References cited:
- EP-A- 1 582 225
- EP-A- 1 586 337
- US-A1- 2005 136 090
- US-A1- 2005 208 093
- KIM WEON ET AL: "The effect of carvedilol-loaded BiodivYsio stent on neointimal hyperplasia in a porcine coronary stent restenosis model." JOURNAL OF THE AMERICAN COLLEGE OF CARDIOLOGY, vol. 41, no. 6 Supplement A, 19 March 2003 (2003-03-19), page 31A, XP002369057 & 52ND ANNUAL SCIENTIFIC SESSION OF THE AMERICAN COLLEGE OF CARDIOLOGY; CHICAGO, IL, USA; MARCH 30-APRIL 02, 2003 ISSN: 0735-1097
- ABIZAID ALEXANDRE ET AL: "First human experience with the 17-beta-estradiol-eluting stent: The Estrogen And Stents To Eliminate Restenosis (EASTER) trial." JOURNAL OF THE AMERICAN COLLEGE OF CARDIOLOGY, vol. 43, no. 6, 17 March 2004 (2004-03-17), pages 1118-1121, XP002369058 ISSN: 0735-1097
- WESSELY RAINER ET AL: "Inhibition of neointima formation by a novel drug-eluting stent system that allows for dose-adjustable, multiple, and on-site stent coating" ARTERIOSCLEROSIS THROMBOSIS AND VASCULAR BIOLOGY, vol. 25, no. 4, April 2005 (2005-04), pages 748-753, XP002369059 ISSN: 1079-5642 cited in the application
- RAJA SHAHZAD G: "Drug-eluting stents: is it the beginning of the end for coronary artery bypass surgery?" CHINESE MEDICAL JOURNAL (ENGLISH EDITION), vol. 117, no. 9, September 2004 (2004-09), pages 1377-1387, XP002369060 ISSN: 0366-6999
- GERSHLICK ANTHONY ET AL: "Inhibition of restenosis with a paclitaxel-eluting, polymer-free coronary stent: The European evaLUation of pacliTaxel Eluting Stent (ELUTES) trial." CIRCULATION, vol. 109, no. 4, 3 February 2004 (2004-02-03), pages 487-493, XP002369061 ISSN: 0009-7322
- LANSKY ALEXANDRA J ET AL: "Non-polymer-based paclitaxel-coated coronary stents for the treatment of patients with de novo coronary lesions: angiographic follow-up of the DELIVER clinical trial." CIRCULATION. 27 APR 2004, vol. 109, no. 16, 27 April 2004 (2004-04-27), pages 1948-1954, XP002369062 ISSN: 1524-4539
- HAUSLEITER JÖRG ET AL: "Prevention of restenosis by a novel drug-eluting stent system with a dose-adjustable, polymer-free, on-site stent coating." EUROPEAN HEART JOURNAL. AUG 2005, vol. 26, no. 15, August 2005 (2005-08), pages 1475-1481, XP002369063 ISSN: 0195-668X

## Description

The present invention relates to an artificial implant or a part thereof coated with at least two drugs, a method of preparing the same and a method of treating a subject, wherein the implant according to the invention is implanted into a subject.

Implants are artificial devices which made to replace or act as a missing biological structure. Additionally or alternatively, implants are used as a depot for substances such as drugs which are to be released to the surrounding tissue. The second type of implant may be used for e.g. brachytherapy, i.e. the placement of a substance such as a radioactive source in or near tissue to deliver e.g. radiation therapy.

Another particular type of implants are stent, which are used on diverse structures such as the oesophagus, trachea, or blood vessels. Prior to use, a stent is in general collapsed to a small diameter; when brought into place it is expanded using an inflatable balloon and is then held in place by its own tension. Stents are usually inserted by endoscopy or other procedures less invasive than a surgical operation, which makes them suitable for patients with advanced disease for whom an operation might be too dangerous. Stents may consist of wire mesh alone, or be covered by a tissue lining.

Coronary stent implants are used for therapeutic cardiac procedures. Re-narrowing of a previously treated vascular lesion, termed restenosis, is considered the most important problem in interventional cardiovascular medicine. Stent placement has been shown to decrease the rate of restenosis. It involves placing a stent, which is a small mesh-like wire tube in a narrowed blood vessel. This procedure is similar to angioplasty in many ways. However, the stent is left permanently in place in the vessel to act as a scaffold to help keep the vessel open. Usually, an angioplasty procedure is performed first. The balloon inflations help open the vessel to allow the stent to be placed easily. The angioplasty catheter is removed. A different angioplasty catheter with a stent crimped on the balloon is advanced into the vessel and carefully positioned at the blockage. The angioplasty balloon is inflated which opens the stent and presses it into the vessel wall. The stent holds the vessel open and helps reduce the rate of restenosis (a recurrence of the narrowing within the vessel). In general the vessel is an artery.

Most of the implants (or at least a part of them) that are used today are made of bare-metal and/or polymeric material. Polymers are known to induce inflammatory responses which can translate into delayed healing and thus increased risk for an adverse outcome.

Furthermore, also bare metal implants are associated with side-effects. It has been shown that up to 40 % of patients who received a bare-metal stent develop an in-stent restenosis.

The risk of adverse reactions or side effects can be reduced by additional administration of suitable drugs preventing the same. For this, implants releasing drugs which reduce adverse reactions or side effects have been developed.

For example, in-stent restenosis can be substantially reduced by the implantation of a drug-eluting stent (DES) (Babapulle et al., 2004, The Lancet 364: 583 - 591). Currently, two devices are approved by the FDA, both of which have shown efficacy towards the prevention of restenosis, the Cypher^{®} stent (Cordis, Johnson&Johnson), which is coated with rapamycin (Moses et al., 2003, N Eng J Med 349: 1315 - 1323) and the Taxus^{®} stent (Boston Scientific) (Stone et al., 2004, N Engl J Med 350: 221 - 231) that releases paclitaxel.

To protract the release of the cytostatic compound, both devices make use of a polymeric coating. While this choice of coating is effective, it imposes several risks which may limit the clinical outcome, since the used polymers are known to induce inflammatory responses as detailed above. In deceased patients who previously received a polymer coated drug-eluting stent, ongoing inflammation and impeded endothelialization could be observed consistently. Additionally, there are concerns that polymer-coated DES implanted in patients may increase the chance for late stent thrombosis as well as late in-stent restenosis by means of chronic adverse pathomechanisms induced by the polymer.

To circumvent the need for polymeric coating, a drug-eluting stent platform that uses a microporous stent surface that serves as "drug pockets" to delay the release of a given drug has been recently introduced (Wessely et al., 2005, Arteriolscler Thromb Vasc Biol 25: 748 - 753). This DES system liberates two thirds of the drug within the first week. The system proved safe and effective both in a standard pre-clinical model as well as in humans.

However, it is believed that the protracted drug release of the polymer-coated stents described above, which liberate the drug within 60 days, is responsible for the clinical results concerning in-stent restenosis at 6-9 months follow up. Therefore, in certain clinical situations a slower release kinetic is required. Modulation of release kinetics of an implant-based, e.g. stent-based, compound to prevent e.g. in-stent restenosis is crucial to improve clinical efficacy. Most current drug-eluting stent platforms use polymeric coating to achieve protracted drug liberation; however, potential life-threatening risks have been identified for polymers, such as late stent thrombosis. Therefore, retardation of drug release from the stent platform has to be achieved by an alternative mechanism.

Accordingly, it is one object of the present invention to provide drug-releasing artificial implants, wherein the drug-release is not controlled by a polymer.

The object is solved by a novel polymer free method of controlling, preferably retarding, the release of the implant-based primary compound by simultaneous addition of a secondary compound. A simultaneous coating (for instance of a microporous stent) by addition of an appropriate secondary drug that leads to control, e.g. protraction, of the release of the primary compound is disclosed. This primary compound or first drug (e.g. rapamycin) is the effector drug to having a medical function. The purpose of the secondary compound or second drug is predominantly to control, e.g. retard, release of the primary drug.

A first aspect of the invention relates to an artificial implant or a part thereof coated with at least two drugs, wherein the release of the first drug is controlled by the second drug, wherein the implant or part thereof is polymer-free and wherein the first drug is rapamycin or an analogue thereof and wherein the second drug is carvedilol, 17β estradiol valerate or probucol.

The potential advantages of multiplex coating are evident: control, e.g. retardation, of primary drug release without the adverse reactions or side effects of polymers controlling the release of drugs. Thus, the proposed multiplex implant coating process and the resulting implant improve both efficacy as well as safety of drug-eluting implants such as stents.

According to the invention, the release of the first drug (primary compound) is controlled by the second drug (secondary compound). With the terms "controlled" or "control" is meant that the release of the first drugs is altered by the second drug, if the implant is coated with both drugs in comparison to the release of the.first drug, if the implant is only coated with the first drugs. In the invention the release of the first drug is protracted by the second drug. The release of the first drug can be determined as detailed in the EXAMPLES (see Examples 1 to 3).

In general, the release of the first drug from the implant may be controlled by the second drug by the following mechanisms:
1. The release may be protracted, if the first drug is attracted by the presence of the second drug on the implant. In this case, there is a higher probability for one molecule of the first drug to stay on the implant coated with the second drug in comparison to an uncoated implant in to the respective surrounding milieu. This will depend on the character of the first and the second drug and on the surrounding milieu. However, the surrounding milieu will be more or less an isotonic salt solution. Therefore, the release of the first drug may be protracted, if the second drug attracts the first drugs, e.g. by chemical properties similar to that of the first drug.
   The first drug may be attracted, if e.g. both drugs
   - share similar chemical structure,
   - have similar lipophilicity,
   - have opposite charges,
   - have similar viscosity,
   - have similar density,
   - develop polar bonds,
   - develop hydrogen bonds,
   - have suitable volume parameters, and/or
   - are attracted by van der Waal's forces.
2. The release may be accelerated, if the first drug is rejected by the implant coated with the second drug on the implant. In this case, there is a higher probability for one molecule of the first drug to leave the implant coated with the second drug and to stay in the respective surrounding milieu. Again, this will depend on the character of the first and the second drug. The release of the first drug may be accelerated, if the second drug rejects the first drugs, e.g. by higher solubility of the first drug in the surrounding milieu or by equal charge of both drugs.

If the release is protracted, the first drug release from multiplex coated implant (coating with first and second drug) preferably has a half-life being at least about 1.5-times the half-life of first drug release of single coated implant (coating with first drug only), more preferably at least about 2-times, even more preferably at least about 3-times, still more preferably at least about 4-times and most preferably at least about 5-times.

If the release is accelerated, the first drug release from multiplex coated implant (coating with first and second drug) preferably has a half-life being at most about 0.9-times the half-life of first drug release of single coated implant (coating with first drug only), more preferably at most about 0.75-times, even more preferably at most about 0.5-times, still more preferably at most about 0.25-times and most preferably at most about 0.1-times.

According to the present invention the first drug is the drug, whose release is controlled, preferably protracted, by the second drug. The first drug is rapamycin, i.e. a substance intended and/or suitable for use in the diagnosis, cure, mitigation, treatment or prevention of disease, disorder, abnormal physical state, or the physical symptoms thereof, in a human or animal receiving the implant.

The purpose of the second drug is predominantly to control, e.g. retard, release of the first drug. Additionally, the secondary compound may impose beneficial medicinal, e.g. vascular effects, yet, this is not mandatory.

According to the invention the artificial implant may be coated with more than two drugs. If more than two drugs are present, the third, forth etc. drug is defined as the (second) drug. However, the third, forth etc. drug may or may not control the release of the first drug.

An artificial implant may be any implant made by man and known in the art. An implant is an artificial device which is made e.g. to replace and/or act as a missing biological structure, to support a deleftive structure or as a depot for a substance. There is a multitude of different implants which replace different functions in humans. Modem medical implants are generally high-end devices. The complete implant or its outer shell is in general made of an bioinert material, in most cases from metal such as titanium. In some cases implants contain electronics e.g. artificial pacemakers and cochlear implant. In other cases the medical implant has compound structure and/or acts as reinforcement e.g. dental implant, knee joint replacement implant. In the context of the present invention the complete implant or anly a part therof may be coated with the first and the second drug.

In a preferred embodiment of the invention the implant is made of metal. Examples of metals suitable for implants include stainless steel, cobalt, chromium, Nickel-titanium shape memory metal Nitinol, and titanium or alloys comprising or consisting of these. Examples of such alloys are titanium- or cobalt/chromium-based alloys. Additionally, other materials may be present such as ceramic materials (aluminium oxide or zirconium oxide) or polymeric materials such as polyethylene, polyglycolic-polylactic acid, polyethyleneoxidepolybutylene, terephthalate, polyorthoester, Biogold polyamine-heparin, methacryloylphosphorylcholine-laurylmethacrylate polycaprolactone, polyethylene terephthalate, silicone and polyurethane.

The implant may be composed of different layers, such as a core, e.g. a ceramic core, and a can, such as titanium can. Additionally, implants may be coated with biological materials such as fibrin, cellulose or albumine. However, implants without non-naturally occurring polymeric surface materials are preferred.

Examples of implants include, without limitation pacemakers or heart valves, metal implants in a patient's brain, eye or ears, dental implants, orthopaedic implants such as hip and knee replacements; plates, screws, and rods used to treat fractures, drug release systems, cavity fillings, orthopaedic devices, stents, self-expanding endoluminal prosthesis sleeves for stent delivery, finger joints, and coronary artery bypass grafts etc.

In one preferred embodiment of the invention the implant is a valve, a dental implant or an orthopaedic implant system.

In another preferred embodiment of the invention the implant is a stent. A stent is a tube made of e.g. metal or plastic that is inserted into a vessel or passage to keep it open and prevent closure due to a stricture or external compression, e.g., to keep an artery open after a heart attack. In an effort to reduce or prevent restenosis of the vessel stents coated according to the invention may be used.

If the implant is a coated stent, any stent known to the skilled practicioner may be used. Preferably the stent is a bare metal stent with or without surface modification. Examples of stents, which are commercially available from e.g. Boston Scientific (Natick, MA, USA), Medtronic Inc. (Minneapolis, MN, USA), Abbott Laboratories (Abbott Park, IL, USA), Guidant Corporation (Indianapolis, IN, USA), Johnson & Johnson (New Brunswick, NJ, USA), Biometric GmbH & Co. KG (Berlin, Germany) or OptiMed Technologies, Inc. (Fairfield, NJ, USA), include - without limitation:
- Bx VELOCITY® Coronary Stent (Johnson & Johnson)
- S.M.A.R.T.® CONTROLTM Stent Delivery System (Johnson & Johnson)
- PRECISE® Nitinol Self-expanding Stent (Johnson & Johnson)
- PALMAZ® CORINTHIAN™ Transhepatic Biliary Stent and Delivery System (Johnson & Johnson)
- Driver™ Coronary Stent (Medtronic)
- Racer Stent (Medtronic)
- Liberté Coronary Stent System (Boston Scientific)
- Magic WALLSTENT® stent (Boston Scientific)

The stent is coated with rapamycin or an analogue thereof as defined below. As detailed above, the coronary artery response to stent implantation leads to a complex and largely predictable sequence of events related to inflammation and repair processes including thrombosis disturbances (acute phase) and tissue remodelling (chronic phase). Interestingly, neither oral anticoagulants, anti-inflammatories, or anti-proliferative drugs have prevented restenosis. Therefore, it is desirable to coat the stent with a first drug having at least one of the following properties:
- Antithrombotic
- Anti-inflammatory
- Anti-proliferative
- Non toxic
- Effect only on multiplying cells
- More or less complete re-endothelialization
- No late thrombosis

In one embodiment of the invention the first drug may be further characterized by its lipophilicity. LogP values (logarithm of partition coeffizient in n-octanol/water) quantifying lipophilicity can be either determined experimentally according to procedures well known to the skilled person or calculated e.g. using a series of computer programs such as HyperChem 7.0, XLOGP 2.0, KowWin, MLOGP, CLOGP etc. Preferably, logP values are calculated using the following formular: logP = log(Co:Cw) where Co is the concentration of compound in the octanol phase and Cw is the concentration in the aqueous phase when the system is at equilibrium (Poole, SW and Poole, CF, J of Chromatogr B Analyt Technol Biomed Life Sci., 797: 3-19,2003).

The first drug may be characterized by a logP value of at least 1.5, more preferably of at least 2.0, even more preferably at least 2.5, still more preferably at least about 3.0, particularly of at least 3.5 and most preferably at least about 4.0.

In the context of the present invention the terms "derivative" and "analogue" are used interchangeably. These terms refer to chemical modifications of the respective basic molecule. Chemical modifications are known to the skilled person and include e.g. hydroxylation, alkylation, esterification, oxidation, hydration, addition or removal of groups such as alkyl, alkenyl, alkinyl, aryl, aralkyl amino, sulfhydryl, hydoxy, fluoro, bromo, carboxy and/or amino groups etc. The basic molecule may be modified in order to maintain or improve the controlling, e.g. protracting, properties of the resulting molecule. The properties can be tested by the test detailed in the EXAMPLES (see Example 1 to 3), in order to determine the resulting kinetics of first drug release.

Rapamycin is a naturally-occurring macrolide antibiotic produced by the fungus *Streptomyces hygroscopicus*, found on Easter Island. The name is derived from Rapa Nui, the native name for Easter Island. Rapamycin is a hydrophobic synthetic drug, FDA-approved as an oral immunosuppressive agent used to prevent organ transplant rejection. Rapamycin (also known as Sirolimus) is an excellent candidate for the treatment of AV graft stenosis, since it significantly reduces in-stent restenosis and prevents chronic organ rejection. It blocks T-cell activation and cytokine and growth factor-mediated smooth muscle cell proliferation. Additionally, Rapamycin does not inhibit the endothelialization of the intima.

Being a hydrophobic drug, it dramatically increases local (vessel wall) concentrations, while allowing exquisite control over release kinetics. Because local drug concentrations are inextricably linked to biological effects, and not mere proximity, rapamycin appears as an ideal anti-restenotic drug. Rapamycin exhibits the following pharmacochemical advantages:
- Cytostatic, not cytotoxic
- Readily diffuses across vascular tissue
- It achieves high local tissue concentration
- Inhibits key target cell cycle
- Long half-life in tissues
- Inhibits inflammation by blocking local cytokines
- Potent (only microgram doses required)
- Safe in humans at blood concentrations far exceeding dose delivered from stents

However, analogues of rapamycin are also useable in the context of the present invention.
Examples include (without limitation) those disclosed in EP1413581 such as: 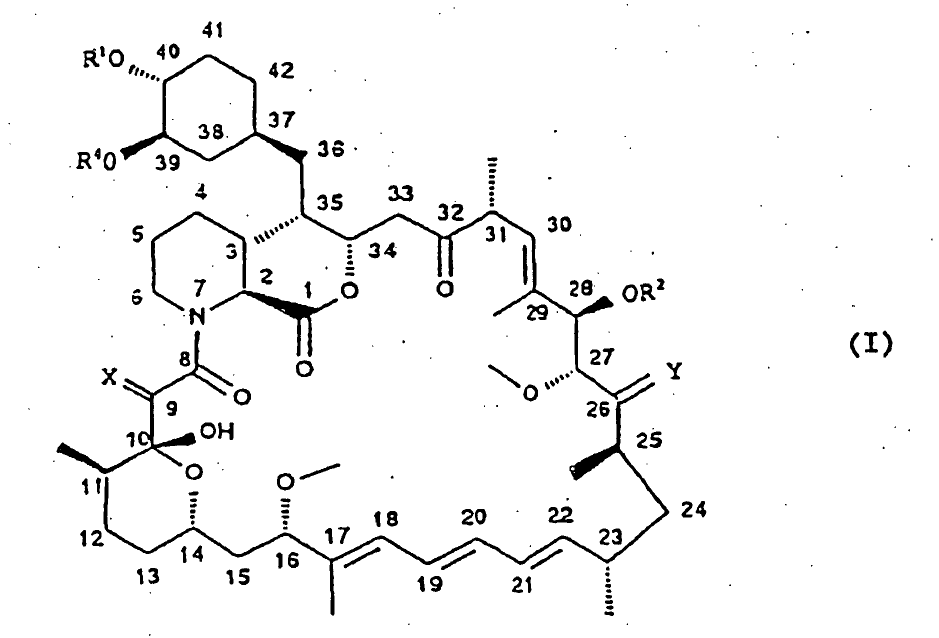 wherein
X is (H,H) or O;
Y is (H,OH) or O;
R¹ and R² are independently selected from H, alkyl, thioalkyl, arylalkyl, hydroxyalkyl, dihydroxyalkyl, hydroxyalkylarylalkyl, dihydroxyalkylarylalkyl, alkoxyalkyl, acyloxyalkyl, aminoalkyl, alkylaminoalkyl, alkoxycarbonylaminoalkyl; acylaminoalkyl, arylsulfonamidoalkyl, allyl, dihydroxyalkylallyl, dioxolanylallyl, carbalkoxyalkyl, and (R³)₃Si where each R³ is independently selected from H, methyl, ethyl, isopropyl, t-butyl; and phenyl; wherein "alk-" or "alkyl" refers to C1-6 alkyl, branched or linear, preferably C1-3 alkyl, in which the carbon chain may be optionally interrupted by an ether (-O-) linkage; and
R⁴ is methyl or R⁴ and R¹ together form C2-6 alkylene;
provided that R¹ and R² are not both H; and
provided that where R¹ is carbalkoxyalkyl or (R³)₃Si, X and Y are not both O.

Preferred examples of these analogues are:
40-O-Benzyl-rapamycin
40-O-(4'-Hydroxymethyl)benzyl-rapamycin
40-O-[4'-(1,2-Dihydroxyethyl)]benzyl-rapamycin
40-O-Allyl-rapamycin
40-O-[3'-(2,2-Dimethyl-1,3-dioxolan-4(S)-yl)-prop-2'-en-1'-yl]-rapamycin
(2'E, 4'S)-40-O-(4',5'-Dihydroxypent-2'-en-1'-yl)-rapamycin
40-O-(2-Hydroxy)ethoxycarbonylmethyl-rapamycin
40-O-(2-Hydroxy)ethyl-rapamycin
40-O-(3-Hydroxy)propyl-rapamycin
40-O-(6-Hydroxy)hexyl-rapamycin
40-O-[2-(2-Hydroxy)ethoxy]ethyl-rapamycin
40-O-[(3S)-2,2-Dimethyldioxolan-3-yl]methyl-rapamycin
40-O-[(2S)-2,3-Dihydroxyprop-1-yl]-rapamycin
40-O-(2-Acetoxy)ethyl-rapamycin
40-O-(2-Nicotinoyloxy)ethyl-rapamycin
40-O-[2-(N-Morpholino)acetoxy]ethyl-rapamycin
40-O-(2-N-Imidazolylacetaxy)ethyl-rapamycin
40-O-[2-(N-Methyl-N'-piperazinyl)acetoxy]ethyl-rapamycin
39-O-Desmethyl-39,40-O,O-ethylene-rapamycin
(26R)-26-Dihydro-40-O-(2-hydroxy)ethyl-rapamycin
28-O-Methyl-rapamycin
40-O-(2-Aminoethyl)-rapamycin
40-O-(2-Acetaminoethyl)-rapamycin
40-O-(2-Nicotinamidoethyl)-rapamycin
40-O-(2-(N-Methyl-imidazo-2'-ylcarbethoxamido)ethyl)-rapamycin
40-O-(2-Ethoxycarbonylaminoethyl)-rapamycin
40-O-(2-Tolylsulfonamidoethyl)-rapamycin
40-O-[2-(4',5'-Dicarboethoxy-1',2',3'-triazol-1'-yl)-ethyl]-rapamycin

Further examples of rapamycin analogues included:
- SDZ RAD (RAD001; 40-O-(2-hydroxyethyl)-rapamycin; everolimus; Novartis International AG, Basel, CH),
- SAR 943 (32-Deoxorapamycin; Sedrani et al., 1998, Transplant Proc 30:2192-2194)
- ABT 578 (zotarolimus; Abbott Laboratories, Abbott Park, IL, USA),
- CCI-779 (temsirolimus; Wyeth-Ayerst Research Laboratories, Princeton, NJ, USA),
- AP20840 (ARIAD Pharmaceuticals, Cambridge, MA, USA)
- AP23573 (ARIAD Pharmaceuticals, Cambridge, MA, USA)
- AP21967 (Pollock et al., 2002, Nature Biotechnology 20, 729 - 733),
- AP22565 (Pollock, R. et al., 2000, Proc. Natl. Acad. Sci. USA 97, 13221-13226),

However, most preferably rapamycin is used as first drug, particularly for cardiovascular implants such as stents.

The second drug may be further characterized by its lipophilicity. LogP values can be determined or calculated as detailed above.

The feature "adrenoreceptor antagonists" relates to a group of substances capable of partially or fully inhibiting adrenergic receptors. Adrenergic receptors (or adrenoceptors) are a class of G protein-coupled receptors that is the target of carecholamines. Adrenergic receptors specifically bind their endogenous ligands, the catecholamines adrenaline and noradrenaline (also called epinephrine and norepinephrine) and are activated by these. The class of adrenergic receptors is subdivided into two subclasses, α- and β-preceptors, wherein each subclass comprises several subtypes (α₁-, α₂-, β₁-, β₂- and β₃-adrenocepor). In general adrenergic blockers mimic the structure of a catecholamines, however, replacing the catechol ring with a bulky substituent (e.g. propranolol) makes it likely that the resulting compound will block adrenoceptors, as the interactions on loop V of the receptor are important to agonist activity.

α-Blockers, also known as α-adrenergic blockers, are used to treat high blood pressure and other conditions like an enlarged prostate. Examples of a blockers are phenoxybenzamine, phentolamine, phenoxybenzamine, prazosin, doxazosin, terazosin, tamsulosin, yohimbine, idazoxan,

β-Blockers, also known as β-adrenergic blockers, are used to treat e.g. high blood pressure (hypertension), congestive heart failure (CHF), abnormal heart rhythms (arrhythmias), and chest pain (angina). β-Blockers are sometimes used in heart attack patients to prevent future heart attacks. They constitute a heterogeneous class of agents having a different degree of selectivity of β₁-adrenergic receptors and the presence of additional properties. Based on these characteristics, β-blocking agents have been categorized into three classes. First generation agents, such as propranolol and timolol, block both β₁- and β₂-receptors and do not exhibit any particular property. These agents are not well tolerated in heart failure because they exhibit negative inotropic effects associated with an increase in peripheral vascular resistance, secondary to β₂-blockade. Second generation agents, such as metoprolol, atenolol, and bisoprolol produce a selective β₁-blockade and do not have additional properties. They do not increase peripheral vascular resistance and are better tolerated by heart failure patients. However, acute administration may produce a decline in cardiac output and an increase in ventricular filling pressure due to negative inotropic effects. Third generation agents, such as bucindolol and carvedilol, are not selective but exhibit ancillary properties, which may be important to their tolerability and efficacy in heart failure patients.

Examples of β-blockers are propranolol, alprenolol, oxprenolol, metoprolol, acebutolol, atenolol, betaxolol, bisoprolol, carteolol, carvedilol, labetalol, nadolol, penbutolol, pindolol, sotalol and timolol.

The second drug may be carvediolol or an analogue thereof. Carvedilol is a vasodilating β-blocker and antioxidant approved for treatment of mild to moderate hypertension, angina, and congestive heart failure. SB 211475, a hydroxylated carvedilol analogue, is an even more potent antioxidant in several assay systems.

Steroids are cholesterol derivatives and have tetracyclic basic structure consisting of four fused rings: three cyclohexane rings and one cyclopentane. Rings A and D of steroids are the most commonly modified rings.

Steroids have a variety of uses in the human body, including, but not limited to: controlling meiosis, carbohydrate metabolism, fat storage, muscle growth, immune function and nerve cell membrane chemistry. Steroids can also be divided into groups by function: androgens, estrogens, estradiol, progestogens, anabolics, and catabolics. The gonadal variety is mainly synthesized in the gonads, as is suggested by the name, while the glucocorticoids (e.g. cortisol, cortisone) and mineral corticoids (eg aldosterone) are synthesized in the adrenal cortex.

Examples of steroids are: cholesterol, estradiol, testosterone, cortisone, cortisol, corticostreone, aldosterone, progesterone, 7-dehydocholesterol, cholic acid, pregnenolone, dihydrotestosterone, nandolone, dihydoandrolone, androdiol, oxandolone and anadrol.

The second drug may be 17β estradiol valerate.

In a further preferred embodiment of the invention the second drug is the antilipemic agent probucol. Antilipemic agents are substances used to treat hyperlipidemia. Examples of antilipemic agents include nicotinic acid, clofibrate, probucol, gemfibrozil, fenofibrate, dextrothyroxine, dexfenfluoramine and lovastatin.

The second drug may be probucol.

The stent according to the present invention and as defined and described above may be used in the prophylaxis or therapy of a stenosis or restenosis.

Another aspect of the present invention relates to a method of preparing the implant according to the present invention defined and described above, wherein an uncoated implant is coated simultaneously or consecutively with the first and the second drug.

The implant may be coated by any procedure suitable for coating a solid material with a solution. The implant may e.g. be dipped into a solution containing both drugs. Alternatively, it can be dipped consecutively in two solutions, wherein one solution contains the first drug and the other the second drug. The concentration of the drug solution is preferably from about 0.1 to about 10 %, more preferably from about 0.2 to about 5 %, still more preferably from about 0.3 to about 3 % and most preferably from about 0.5 to about 1 %.

Thereafter, the implant is dried e.g. by mild heating, air-drying or any other suitable method known to the artisan. Eventually, the implant may be implanted into a subject, such as a human or an animal.

In a preferred embodiment of the invention the uncoated implant is spray-coated. Even more preferably, the coating machine described by Wessely and coworkers is used, which permits individual, on-site coating of implants such as stents e.g. with a unique microporous surface (Wessely R. et al., 2005, Arterioscler Thromb Vasc Biol 25: 748 - 753) allowing for individualizable, dose-adjustable, and multiple coatings with identical or various compounds, designated ISAR (individualizable drug-eluting stent system to abrogate restenosis). Preferably, stents can be coated using the drug solutions as defined above. Most preferably, the implant suach as a stent is coated with rapamycin and a second drug such as carvedilol or 17β estradiol valerate or an analogue thereof.

The description also provides a method of treating a subject, wherein an implant, preferably a stent, according the present invention and as defined and described above is implanted into a subject.

Methods of implanting an implant into a subject such a human or an animal are known to the skilled practitioner.

If a stent is to be implanted, the following procedure may be carried out: the stent is collapsed to a small diameter and put over a balloon catheter. It is then moved into the area of the blockage. When the balloon is inflated, the stent expands, locks in place and forms a scaffold. This holds the vessel open. The stent stays in the vessel permanently, holds it open, improves blood flow to the heart muscle and relieves symptoms (usually chest pain).

### FIGURES

**Fig. 1** shows retardation of stent-based rapamycin release by addition of carvedilol 1% for spray coating. Top panel shows release curve, bottom panel total remaining rapamycin on the stent at the end of the observational period.
   • Rapamycin 1 %
   o Rapamycin 1% + Carvedilol 0.5%
**Fig. 2** shows retardation of stent-based rapamycin release by addition of estradiolvalerat 1% for spray coating. Top panel shows release curve, bottom panel total remaining rapamycin on the stent at the end of the observational period.
   • Rapamycin 1%
   o Rapamycin 1% + Estradiolvalerat 0.5%
**Fig. 3** shows no retardation of stent-based rapamycin release by addition of rosiglitazone 0.5% for spray coating. Top panel shows release curve, bottom panel total remaining rapamycin on the stent at the end of the observational period.
   • Rapamycin 1%
   o Rapamycin 1% + Rosiglitazone 0.5%
Fig. 4 shows the release profile of stent coating solutions containing 4% rapamycin (R4%) and various concentrations of probucol (Pro) (0.1 %Pro, 0.5%Pro and 1%Pro relates to 0.1%, 0.5% and 1.0% probucal, respectively). Rapamycin release from the stent platform is modifiable by usage of appropriately concentrated probucol.
   ◆ Rapamycin 4% + Probucol 0.1%
   ■ Rapamycin 4% + Probucol 0.5%
   ▲ Rapamycin 4% + Probucol 1.0%
   × Rapamycin 4%

### EXAMPLES

### Example 1: Effects of carvedilol on the release profile of rapamycin

### Experimental procedure:

Microporous stents are spray-coated by means of a unique stent-coating machine (Wessely et al, supra). Attachment of a drug-reservoir is required to supply one or multiple drugs of choice. Besides sirolimus (rapamycin) we included a second drug at different concentrations to examine whether release kinetics of the primary compound (e.g. sirolimus) could be altered. Released rapamycin from the stent platform were measured at distinctive time points as previously described (Wessely, supra) by means of UV spectroscopy at a wave length of 280 nm. Beforehand, it was confirmed that the UV spectres of the secondary drugs do not interfere with the one from rapamycin.

### Results:

To prove principle, we added *carvedilol*, a drug commonly used to treat hypertension and heart failure to the standard dosage of rapamycin (1%).

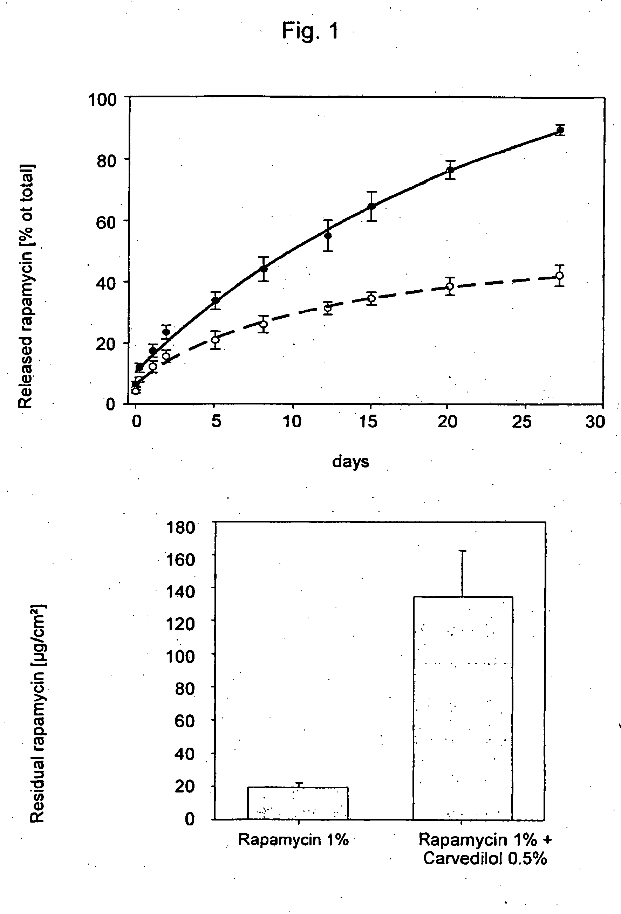

To test whether carvedilol is able to modulate rapamycin release, the release of single coated rapamycin (1%) was compared to simultaneous multiplex coating with a solution containing both rapamycin (1%) and carvedilol (0.5%), dissolved in ethanol. The result is shown in Fig.1 with a clear retardation of stent-based rapamycin by carvedilol compared to rapamycin only coated stents.

### Example 2: Effects of different concentrations of 17β estradiol valerate on the release profile of rapamycin

### Experimental procedure:

The experiment was carried out as Example 1 with the exception that 17β estradiol was used as second drug.

### Results:

To further prove principle, we added 17β estradiol valerate, a hormone that imposes several vascular protective effects, to the standard dosage of rapamycin (1%).

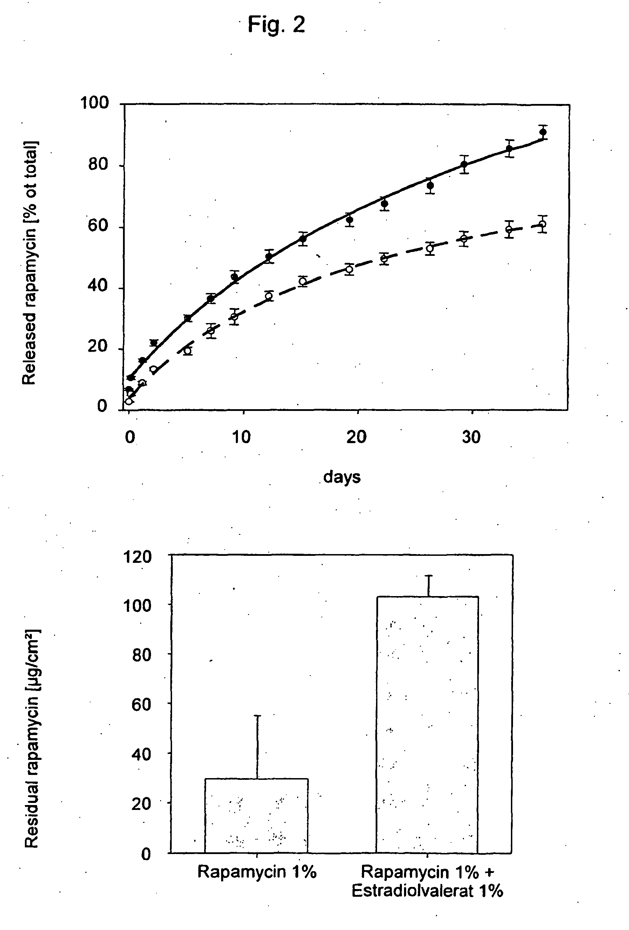

Analogously to the previous coating experiment, we tested the impact of different concentrations of 17β estradiol valerate on the release profile of a 1% rapamycin solution, dissolved in ethanol. 0.1%, 0.5% as well as 1.0% 17β estradiol valerate were added to the solution containing rapamycin at a final concentration of 1%. All investigated concentrations were able to substantially modulate rapamycin release from the stent platform (Fig. 2).

### Comparative Example 3: Effects of rosiglitazone on the release profile of rapamycin

### Experimental procedure:

The experiment was carried out as Example 1 with the exception that rosiglitazone was used as second drug.

### Results:

To further prove principle, we added rosiglitazone, a PPARγ ligand that is currently used to treat type II diabetes mellitus but also imposes protective effects on the vasculature.

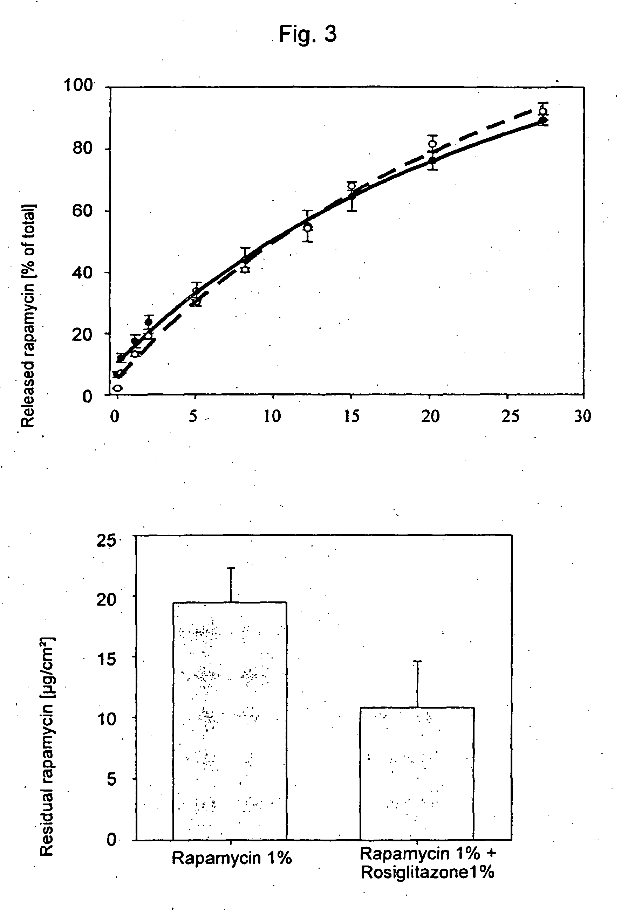

In this case, the effect of a 0.5% solution in conjunction with rapamycin 1% in ethanol was compared to the release of 1% rapamycin alone. Divergent to the previous observations with carvedilol and 17β estradiol valerate there was no detectable modulatory effect regarding the release kinetics of rapamycin (Fig. 3).

### Example 4: Effects of different concentrations of probucol on the release profile of rapamycin

### Experimental procedure:

The experiment was carried out as Example 1 with the exception that probucol was used as second drug.

### Results:

To further prove principle, we added probucol, a drug that imposes primarily antilipemic and anti-oxidative properties, to a dosage of rapamycin of 4%. Additionally, this drug comprises additional anti-restenotic properties.

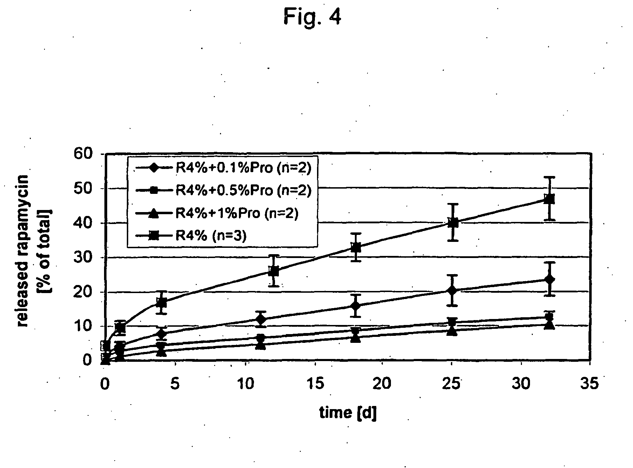

Analogously to the previous coating experiment, we tested the impact of different concentrations of probucol on the release profile of a 4% rapamycin solution, dissolved in ethanol. 0.1%, 0.5% as well as 1.0% probucol were added to the solution containing rapamycin at a final concentration of 4%. Combined coating of a stent with rapamycin and probucol protracts the release of rapamycin without the use of a polymer by physicochemical interactions (Fig. 4). Importantly, rapamycin release could be modified by the concentration of probucol that was simultaneously applied for stent coating.

Additionally, microscopic analysis of the abluminal surface of a drug-eluting stent coated with a rapamycin/probucol solution (1.0% rapamycin and 1.0% probucol) suggested that the abluminal surface is covered appropriately and smoothly by the coating solution. Appearance of the stent surface after coating with a solution that was comprised of shows an appropriate appearance (data not shown).

## Claims

1. An artificial implant or a part thereof coated with at least two drugs, wherein the release of the first drug is controlled by the second drug, wherein the implant or part thereof is polymer-free, wherein the first drug is rapamycin or an analogue thereof and wherein the second drug is carvedilol, 17β estradiol valerate or probucol.

2. The implant or part thereof of claim 1, wherein the release of the first drug is protracted by the second drug.

3. The implant or part thereof of any of claims 1 or 2, wherein the implant is made of metal.

4. The implant or part thereof of any of claims 1 to 3, wherein the implant is a valve, a dental implant or an orthopaedic implant system.

5. The implant of any of claims 1 to 3, wherein the implant is a stent.

6. The implant or part thereof of any of claims 1 to 5, wherein the first drug is **characterized by** a logP value of at least 1.5, more preferably of at least 2.0, even more preferably at least 2.5, still more preferably at least about 3.0, particularly of at least 3.5 and most preferably at least about 4.0.

7. The implant of any of claims 1 to 6, wherein the second drug is **characterized by** a logP value of from 1.5 to 8, more preferably from 2 to 7, even more preferably from 2.5 to 6.5 and still more preferably from 3 to 6.

8. A method of preparing the implant according to any of claims 1 to 7, wherein an uncoated implant is coated simultaneously or consecutively with the first and the second drug.

9. The method of claim 8, wherein the uncoated implant is spray-coated.

10. The implant of claim 5 for use in the prophylaxis or therapy of a stenosis or restenosis.

## Patentansprüche

1. Künstliches Implantat oder Teil davon, beschichtet mit mindestens zwei Arzneimitteln, wobei die Freisetzung des ersten Arzneimittels durch das zweite Arzneimittel kontrolliert ist, wobei das Implantat oder der Teil davon polymerfrei ist, wobei das erste Arzneimittel Rapamycin oder ein Analogon davon ist, und wobei das zweite Arzneimittel Carvedilol, 17β-Estradiolvalerat oder Probucol ist.

2. Implantat oder Teil davon nach Anspruch 1, wobei die Freisetzung des ersten Arzneimittels durch das zweite Arzneimittel protrahiert ist.

3. Implantat oder Teil davon nach einem beliebigen der Ansprüche 1 oder 2, wobei das Implantat aus Metall hergestellt ist.

4. Implantat oder Teil davon nach einem beliebigen der Ansprüche 1 bis 3, wobei das Implantat ein Ventil, ein Zahnimplantat oder ein orthopädisches Implantatsystem ist.

5. Implantat nach einem beliebigen der Ansprüche 1 bis 3, wobei das Implantat ein Stent ist.

6. Implantat oder Teil davon nach einem beliebigen der Ansprüche 1 bis 5, wobei das erste Arzneimittel durch einen logP-Wert von mindestens 1,5, stärker bevorzugt von mindestens 2,0, noch stärker bevorzugt mindestens 2,5, noch stärker bevorzugt mindestens etwa 3,0, insbesondere von mindestens 3,5 und am stärksten bevorzugt mindestens etwa 4,0 **gekennzeichnet** ist.

7. Implantat nach einem beliebigen der Ansprüche 1 bis 6, wobei das zweite Arzneimittel durch einen logP-Wert von 1,5 bis 8, stärker bevorzugt 2 bis 7, noch stärker bevorzugt 2,5 bis 6,5 und noch stärker bevorzugt 3 bis 6 **gekennzeichnet** ist.

8. Verfahren zur Herstellung des Implantats gemäß einem beliebigen der Ansprüche 1 bis 7, wobei ein unbeschichtetes Implantat simultan oder konsekutiv mit dem ersten und dem zweiten Arzneimittel beschichtet wird.

9. Verfahren nach Anspruch 8, wobei das unbeschichtete Implantat sprühbeschichtet wird.

10. Implantat gemäß Anspruch 5 zur Verwendung in der Prophylaxe oder Therapie einer Stenose oder Restenose.

## Revendications

1. Implant artificiel ou partie de celui-ci revêtu d'au moins deux substances médicamenteuses, dans lequel la libération de la première substance médicamenteuse est commandée par la seconde substance médicamenteuse, dans lequel l'implant ou partie de celui-ci est dépourvu de polymère, dans lequel la première substance médicamenteuse est de la rapamycine ou une substance analogue à celle-ci, et dans lequel la seconde substance médicamenteuse est du carvédilol, du 17β estradiol valérate ou du probucol.

2. Implant ou partie de celui-ci selon la revendication 1, dans lequel la libération de la première substance médicamenteuse est prolongée par la seconde substance médicamenteuse.

3. Implant ou partie de celui-ci selon l'une quelconque des revendications 1 ou 2, dans lequel l'implant est réalisé en métal.

4. Implant ou partie de celui-ci selon l'une quelconque des revendications 1 à 3, dans lequel l'implant est une valve, un implant dentaire ou un système d'implant orthopédique.

5. Implant selon l'une quelconque des revendications 1 à 3, dans lequel l'implant est une endoprothèse vasculaire.

6. Implant ou partie de celui-ci selon l'une quelconque des revendications 1 à 5, dans lequel la première substance médicamenteuse est **caractérisée par** une valeur logP d'au moins 1,5, plus préférentiellement d'au moins 2,0, encore plus préférentiellement d'au moins 2,5, beaucoup plus préférentiellement d'au moins 3,0, particulièrement d'au moins 3,5, et le plus préférentiellement d'au moins 4,0.

7. Implant selon l'une quelconque des revendications 1 à 6, dans lequel la seconde substance médicamenteuse est **caractérisée par** une valeur logP de 1,5 à 8, plus préférentiellement de 2 à 7, encore plus préférentiellement de 2,5 à 6,5, et beaucoup plus préférentiellement de 3 à 6.

8. Procédé de préparation de l'implant selon l'une quelconque des revendications 1 à 7, dans lequel un implant non revêtu est revêtu simultanément ou consécutivement de la première substance médicamenteuse et de la seconde substance médicamenteuse.

9. Procédé selon la revendication 8, dans lequel l'implant non revêtu est revêtu par pulvérisation.

10. Implant selon la revendication 5, prévu pour utilisation dans la prophylaxie ou la thérapie d'une sténose ou d'une resténose.
